# EUROPEAN PATENT SPECIFICATION

(11) **EP 0 625 899 B1**
(45) Date of publication and mention of the grant of the patent: **29.05.1996**
(21) Application number: 93917397.7
(22) Date of filing: 04.02.1993
(51) Int. Cl.: A61K 9/127

(54) **LIPOSOMAL ITRACONAZOLE FORMULATIONS**
LIPOSOMALE ITRACONAZOL FORMULIERUNGEN
FORMULATIONS D'ITRACONAZOLE LIPOSOMIQUES

(30) Priority: 12.02.1992 EP 92200397
(43) Date of publication of application: 30.11.1994
(73) Proprietor: JANSSEN-CILAG S.p.A., 20093 Cologno Monzese - Milan (IT)
(72) Inventor: LUCCHETTI, Giovanni, I-00128 Rome (IT); ASSOGNA, Giuseppe, I-00142 Rome (IT); BAGNATO, Pietro, I-04100 Latina (IT)
(74) Representative: Wante, Dirk
(86) International application number: EP9300273
(87) International publication number: WO9315719

(56) References cited:
- EP-A- 0 215 423
- EP-A- 0 230 226
- EP-A- 0 253 619
- EP-A- 0 427 582
- WO-A-88/07871
- WO-A-89/03679

## Description

The present invention relates to a topical liposomal formulation comprising itraconazole, a phospholipid, a solvent system for itraconazole and said phospholipid, water and conventional auxiliary formulating agents, and a method of preparing said formulation.

WO 8903-679-A discloses classic liposome compositions comprising antimycotic agents; similarly JP-2204-413-A discloses classic liposome compositions with fungicides as active ingredient such as antimycotic agents. EP-0,253,619 describes a convenient method for preparing single bilayered liposomes containing encapsulated active ingredients such as antimycotic agents.

Itraconazole is a antifungal drug which is sparingly soluble in aqueous and/or alcoholic media. This property has hitherto effectively precluded the development of efficacious topical itraconazole formulations. This problem has now been solved by using an especially designed organic solvent system.

The present liposomal itraconazole formulation penetrates rapidly and deeply in epithelial tissues and shows excellent retention of the active ingredient therein. The efficacy of the composition for treating topical fungal infections is superb. The present formulation is stable and in particular does not show the disadvantage of crystallization of the sparingly soluble active ingredient itraconazole.

The present invention provides a topical liposomal formulation comprising itraconazole, a phospholipid, a solvent system for itraconazole and said phospholipid, water and conventional auxiliary formulating agents, characterized in that said composition comprises as a solvent system dimethylisosorbide and tetraglycol. Both the liposomal formulations of itraconazole and the use of said solvent system in topical liposomal formulations is novel.

The present composition is preferably in the form of single bilayered liposomes, in particular liposomes having a size ranging from 100 to 1000 nm, more in particular from 100 to 500 nm. In general, the stability of the liposomal formulation increases as the size of the single bilayered liposomes decreases. Single bilayered liposomes, also referred to as unilamellar vesicles, consist of a single bilayer of suitable amphophilic molecules such as phospholipids which encapsulate an aqueous phase and which are separated from each other by an aqueous phase.

Hereinafter, the amounts of each of the ingredients in the present composition are expressed as percentages by weight based on the total weight of the formulation. Similarly, ratios are intended to define weight-by-weight ratios.

Suitable phospholipids for use in the present composition are, for example, phosphatidyl cholines, ethanolamines and serines, sphingomyelins, cardiolipins, plasmalogens, phosphatidic acids and cerebrosides, in particular those which are soluble together with itraconazole in non-toxic, pharmaceutically acceptable organic solvents. Preferred phospholipids are, for example, phosphatidyl choline, phosphatidyl ethanolamine, phosphatidyl serine, phosphatidyl inositol, lysophosphatidyl choline, phosphatidyl glycerol and the like, and mixtures thereof especially lecithin, e.g. soya lecithin. The amount of phospholipid used in the present formulation can range from about 10 to about 30%, preferably from about 15 to about 25% and in particular is about 20%.

Lipophilic additives may be employed advantageously to modify selectively the characteristics of the liposomes. Examples of such additives include, for example, stearylamine, phosphatidic acid, tocopherol, cholesterol, cholesterol hemisuccinate and lanolin extracts; cholesterol being a lipophilic additive of choice. The amount of lipophilic additive used can range from 0.5 to 8%, preferably from 1.5 to 4% and in particular is about 2%. Generally, the ratio of the amount of lipophilic additive to the amount of phospholipid ranges from about 1:8 to about 1:12 and in particular is about 1:10.

Said phospholipid, lipophilic additive and the active ingredient itraconazole are employed in conjunction with a non-toxic, pharmaceutically acceptable organic solvent system which can dissolve said ingredients. The organic solvent system used in the present formulation is of crucial importance. Said solvent system not only must dissolve the active ingredient itraconazole completely, but it also has to allow the formulation of stable single bilayered liposomes. The solvent system comprises dimethylisosorbide and tetraglycol (glycofurol, tetrahydrofurfuryl alcohol polyethylene glycol ether) in an amount of about 8 to about 30%, in particular about 12 to about 20% and preferably about 15%. In said solvent system, the ratio of the amount of dimethylisosorbide to the amount of tetraglycol can range from about 2:1 to about 1:3, in particular from about 1:1 to about 1:2.5 and preferably is about 1:2. The amount of tetraglycol in the final composition thus can vary from 5 to 20%, in particular from 5 to 15% and preferably is approximately 10%. The amount of dimethylisosorbide in the final composition thus can range from 3 to 10%, in particular from 3 to 7% and preferably is approximately 5%.

The term "organic component" as used hereinafter refers to mixtures comprising said phospholipid, lipophilic additives and organic solvents.

The active ingredient itraconazole is dissolved in the organic component. It may he advantageous to use micronized forms of the active ingredient to facilitate its dissolution. The amount of active ingredient in the final formulation ranges from 0.1 to 0.6%, in particular from 0.4 to 0.5% and preferably is approximately 0.5%. In addition, other ingredients such as anti-oxidants may be added to the organic component. Examples thereof include tocopherol, butylated hydroxyanisole, butylated hydroxytoluene, ascorbyl palmitate, ascorbyl oleate and the like.

The aqueous component of the present formulation comprises mainly water and may contain various additives such as electrolytes, buffer systems, preservatives and the like. Suitable electrolytes include metal salts, in particular alkali metal and earth alkaline metal salts such as, for example, calcium chlorides, sodium chloride, potassium chloride, preferably sodium chloride. The concentration of the electrolytes may vary over a wide range and depends on the nature and the concentration of each of the ingredients in the final formulation and should he sufficient to stabilize the liposomal membranes. In the present composition the amount of sodium chloride can range from 0.05 to 0.2% and in particular is about 0.1%. Buffer systems comprise mixtures of appropriate amounts of an acid such as phosphoric, succinic, or preferably citric acid, and a base, in particular sodium hydroxide. Said buffer systems should maintain the pH of the formulation within the range of 5 to 7, preferably within the range of 5 to 6.5 and especially within the range of 5.5 to 6. Preservatives which can be employed in the present composition to prevent degradation by microorganisms comprise benzoic acid, methylparaben and propylparaben. Preferably, methylparaben and propylparaben are used in an amount of approximately 0.2 % and 0.02 % respectively. It is especially advantageous to add to the aqueous component a chelating agent such as sodium or disodium edetate in order to prevent the oxidative degradation of the phospholipid in the final formulation by metal ions. An effective amount of for example disodium edetate ranges from 0.05 % to 0.25 % and in particular is approximately 0.15 %.

Preferred compositions comprise by weight based on the total weight of the composition:
(a) 0.1 to 0.6% itraconazole;
(b) 10 to 30% phospholipid;
(c) 0.5 to 8% cholesterol;
(d) 3 to 10% dimethylisosorbide;
(e) 5 to 20% tetraglycol;
(f) buffer to maintain the pH of the composition within the range of 5 to 7;
(g) sufficient electrolytes to stabilize the liposomal membranes;
(h) sufficient dermatologically acceptable preservatives to prevent degradation of the composition;
(i) 0.5 to 1.5% thickening agent; and
(j) water.

Particularly preferred compositions are those wherein:
the amount of itraconazole is 0.4 to 0.5%;
the amount of phospholipid is 15 to 25%;
the amount of cholesterol is 1.5% to 4%;
the amount of dimethylisosorbide is 3 to 7%;
the amount of tetraglycol is 5 to 15%; and
the amount of thickening agent is 1%.

The most preferred composition comprises approximately:
(a) 0.5% itraconazole;
(b) 20% phospholipid;
(c) 2% cholesterol;
(d) 5% dimethylisosorbide;
(e) 10% tetraglycol;
(f) 0.06% sodium hydroxide and 0.1% citric acid;
(g) 0.1% sodium chloride;
(h) 0.2% methylparaben, 0.02% propylparaben, 0.01% butylated hydroxytoluene, and 0.15% disodium edetate;
(i) 1% hydroxypropyl methylcellulose; and
(j) water.

The liposomal itraconazole formulation of the present invention can preferably be prepared by
(a) heating the phospholipid and the organic solvent system to about 60 to 80°C, preferably to about 70 to 75°C in a first vessel, dissolving the active ingredient therein, adding thereto the auxiliary formulating agents, and stirring the mixture until complete dissolution is obtained;
(b) heating part of the water to 90 - 95°C in a second vessel and dissolving the preservatives therein, allowing the mixture to cool and then adding the remainder of the auxiliary formulating agents and the remainder of the water, and stirring the mixture until complete dissolution is obtained; thus preparing the aqueous component;
(c) transferring the contents of the first vessel by means of a vacuum directly into the aqueous component, while homogenizing the thus obtained combination with a high performance mixing apparatus, in particular a high-shear mixer; and
(d) adding a thickener to the resulting mixture by means of a vacuum while further homogenizing.

Preferably, the aqueous component is placed in a suitable vessel which can he equiped with a homogenizer and homogenization is effected by creating great turbulence during the injection of the organic component. Any mixing means or homogenizer which exerts high shear forces on the mixture may be employed. Generally, a mixer capable of speeds from about 1,500 to 20,000 rpm, in particular from about 3,000 to about 6,000 rpm may be employed. Suitable thickening agents for use in process step (d) are for example, xanthan gum, hydroxypropyl cellulose, hydroxypropyl methylcellulose or mixtures thereof, cellulose derivatives being preferred. The amount of thickening agent depends on the nature and the concentration of the other ingredients and in general ranges from about 0.5 to 1.5%, and in particular is approximately 1%.

In order to prevent degradation of the materials used during the preparation of the liposimal formulation, it is advantageous to purge all solutions with an inert gas such as nitrogen or argon, and to conduct all steps under an inert atmosphere.

Liposomes prepared by the above described method usually contain most of the active ingredient bound in the lipid bilayer and separation of the liposomes from unencapsulated material is not required. The single bilayered liposomes can conveniently be employed directly or they may also be employed in combination with other suitable pharmaceutically acceptable carriers for topical administration.

The above described liposomal compositions are particularly useful for the topical treatment of subjects suffering from topical fungal infections, in particular for treating acute forms of said problems. The itraconazole containing compositions are applied topically to the area to be treated at regular intervals, as needed, generally once, twice or thrice a day. The duration of the treatment will depend on the nature, extent and severity of the condition to be treated as well as the frequency of applying the composition.

The following examples are intended to illustrate the scope of the present invention in all its aspects and not to limit it thereto.

### Example 1

| Ingredient | Quantity, mg/g of cream |
|---|---|
| itraconazole | 5.0 |
| soya lecithin | 200.0 |
| cholesterol | 20.0 |
| tetraglycol | 100.0 |
| dimethylisosorbide | 50.0 |
| methylparaben | 2.0 |
| propylparaben | 0.2 |
| butylated hydroxytoluene | 0.1 |
| disodium edetate | 1.5 |
| sodium chloride | 1.0 |
| hydroxypropyl methylcellulose | 10.0 |
| sodium hydroxide | 0.6 |
| citric acid | 1.0 |
| purified water, USP | 608.6 |

### Procedure

1. Heat the soya lecithin, tetraglycol and dimethyl isosorbide to about 70-75°C. Dissolve the itraconazole, cholesterol and butylated hydroxytoluene in the heated mixture. Stir until complete dissolution is obtained.
2. Heat about one third of the water to 80-95°C in a separate vessel and dissolve the preservatives methylparaben and propylparaben in the heated water while stirring. Allow the solution to cool to about 25°C and then add the disodium edetate, sodium chloride, sodium hydroxide and citric acid. Add the remainder of the water and stir to obtain a complete solution.
3. Transfer the mixture resulting from step 1 into the mixture resulting from step 2 by means of a vacuum, while homogenizing the combination with a high-shear mixer until a homogeneous product is obtained.
4. Add the hydroxypropyl methylcellulose into the mixture resulting from step 3 by means of a vacuum while homogenizing with a mixer.

The homogenizer was a Silverson high-shear mixer operating at approximately 3000 rpm. The flow rate during the transfer steps was about 0.5 l/min. Single bilayered liposomes were forms The white lipogel cream resulting from step 4 can conveniently be filled in suitable containers such as tubes, e.g. PVC-covered aluminum tubes.

## Claims

1. A topical liposomal composition comprising itraconazole, a phospholipid, a solvent system for itraconazole and said phospholipid, water and conventional auxiliary formulating agents, characterized in that said composition comprises as a solvent system dimethylisosorbide and tetraglycol.

2. A composition according to claim 1 wherein the liposomes are single bilayered liposomes.

3. A composition according to claim 2 wherein the phospholipid is lecithin.

4. A composition according to claim 3 wherein the amount of dimethylisosorbide and tetraglycol ranges from 8 to 30% by weight based on the total weight of the composition.

5. A composition according to claim 4 wherein the ratio of the amount of dimethylisosorbide to the amount of tetraglycol ranges from 2:1 to 1:3.

6. A composition according to claim 1 comprising by weight based on the total weight of the composition:
(a) 0.1 to 0.6% itraconazole;
(b) 10 to 30% phospholipid;
(c) 0.5 to 8% cholesterol;
(d) 3 to 10% dimethylisosorbide;
(e) 5 to 20% tetraglycol;
(f) buffer to maintain the pH of the composition within the range of 5 to 7;
(g) sufficient electrolytes to stabilize the liposomal membranes;
(h) sufficient dermatologically acceptable preservatives to prevent degradation of the composition;
(i) 0.5 to 1.5% thickening agent; and
(j) water.

7. A composition according to claim 6 wherein
the amount of itraconazole is 0.4 to 0.5%;
the amount of phospholipid is 15 to 25%;
the amount of cholesterol is 1.5% to 4%;
the amount of dimethylisosorbide is 3 to 7%;
the amount of tetraglycol is 5 to 15%; and
the amount of thickening agent is 1%.

8. A composition according to claim 7 comprising approximately;
(a) 0.5% itraconazole;
(b) 20% phospholipid;
(c) 2% cholesterol;
(d) 5% dimethylisosorbide;
(e) 10% tetraglycol;
(f) 0.06% sodium hydroxide and 0.1% citric acid;
(g) 0.1% sodium chloride;
(h) 0.2% methylparaben, 0.02% propylparaben, 0.01% butylated hydroxytoluene, and 0.15% disodium edetate;
(i) 1% hydroxypropyl methylcellulose; and
(j) water.

9. A process for preparing a composition as claimed in any one of claims 1 to 8 characterized in that said process comprises the steps of:
(a) heating the phosholipid and the organic solvent system in a first vessel, dissolving the active ingredient therein, adding thereto the auxiliary formulating agents, and stirring until complete dissolution is obtained;
(b) heating part of the water in a second vessel, dissolving the preservatives therein, allowing the mixture to cool and then adding the remainder of the auxiliary formulating agents and the remainder of the water, and stirring until complete dissolution is obtained, thus preparing the aqueous component;
(c) transferring the contents of the first vessel by means of a vacuum directly into the aqueous component, while homogenizing with a high performance mixer; and
(d) adding a thickener to the resulting mixture by means of a vacuum while further homogenizing.

## Patentansprüche

1. Topische liposomale Zusammensetzung mit einem Gehalt an Itraconazol, einem Phospholipid, einem Lösungsmittelsystem für Itraconazol und das genannte Phospholipid, Wasser und üblichen Formulierungshilfmitteln, dadurch gekennzeichnet, daß die Zusammensetzung als Lösungsmittelsystem Dimethylisosorbid und Tetraglycol umfaßt.

2. Zusammensetzung nach Anspruch 1, worin die Liposomen einzelne Zweischichtliposomen sind.

3. Zusammensetzung nach Anspruch 2, worin das Phospholipid Lecithin ist.

4. Zusammensetzung nach Anspruch 3, worin die Menge an Dimethylisosorbid und Tetraglycol im Bereich von 8 bis 30 Gewichtsprozent, bezogen auf das Gesamtgewicht der Zusammensetzung,liegt.

5. Zusammensetzung nach Anspruch 4, worin das Verhältnis der Dimethylisosorbidmenge zur Tetraglycolmenge von 2:1 bis 1:3 beträgt.

6. Zusammensetzung nach Anspruch 1, umfassend, bezogen auf das Gesamtgewicht der Zusammensetzung:
(a) 0,1 bis 0,6 Gew.-% Itraconazol;
(b) 10 bis 30 Gew.-% Phospholipid;
(c) 0,5 bis 8 Gew.-% Cholesterin;
(d) 3 bis 10 Gew.-% Dimethylisosorbid;
(e) 5 bis 20 Gew.-% Tetraglycol;
(f) Puffer zur Aufrechterhaltung des pH-Wertes der Zusammensetzung im Bereich von 5 bis 7;
(g) genügend Elektrolyte zum Stabilisieren der Liposomalmembranen;
(h) ausreichend dermatologisch annehmbare Konservierungsmittel zur Vermeidung eines Abbaus der Zusammensetzung;
(i) 0,5 bis 1,5 Gew.-% Verdickungsmittel; und
(j) Wasser.

7. Zusammensetzung nach Anspruch 6, worin
die Itraconazolmenge 0,4 bis 0,5% beträgt;
die Phospholipidmenge 15 bis 25% beträgt;
die Cholesterinmenge 1,5 bis 4% beträgt;
die Dimethylisosorbidmenge 3 bis 7% beträgt;
die Tetraglycolmenge 5 bis 15% beträgt; und
die Menge an Verdickungsmittel 1% beträgt.

8. Zusammensetzung nach Anspruch 7, mit einem annähernden Gehalt an:
(a) 0,5% Itraconazol;
(b) 20% Phospholipid;
(c) 2% Cholesterin;
(d) 5% Dimethylisosorbid;
(e) 10% Tetraglycol;
(f) 0,06% Natriumhydroxid und 0,1% Zitronensäure;
(g) 0,1% Natriumchlorid;
(h) 0,2% Methylparaben, 0,02% Propylparaben, 0,01% butyliertem Hydroxytoluol und 0,15% Dinatriumedeteat;
(i) 1% Hydroxypropylmethylcellulose; und
(j) Wasser.

9. Verfahren zur Herstellung einer Zusammensetzung nach einem der Ansprüche 1 bis 8, dadurch gekennzeichnet, daß dieses Verfahren die folgenden Stufen umfaßt:
(a) Erwärmen des Phospholipids und des organischen Lösungsmittelsystems in einem ersten Gefäß, Auflösen des wirksamen Bestandteiles darin, Zusetzen der Formulierungshilfsmittel und Rühren, bis eine vollständige Auflösung erzielt ist;
(b) Erwärmen eines Teiles des Wassers in einem zweiten Gefäß, Auflösen der Konservierungmittel darin, Abkühlenlassen des Gemisches und anschließendes Zusetzen der restlichen Formulierungshilfsmittel und des restlichen Wassers und Rühren bis zur Erzielung einer vollständigen Auflösung, unter Ausbildung der wässrigen Komponente;
(c) Überführen des Inhalts aus dem ersten Gefäß mittels Vakuum direkt in die wässrige Komponente, unter Homogenisieren mit einem Hochleistungsmischer;
(d) Zusetzen eines Verdickungsmittels zu dem gebildeten Gemisch mittels Vakuum unter weiterem Homogenisieren.

## Revendications

1. Composition liposomique topique comprenant de l'itraconazole, un phospholipide, un système solvant de l'itraconazole et dudit phospholipide, de l'eau et des agents auxiliaires classiques de formulation, caractérisée en ce que ladite composition comprend comme système solvant du diméthylisosorbide et du tétraglycol.

2. Composition selon la revendication 1, dans laquelle les liposomes sont des liposomes à une bicouche.

3. Composition selon la revendication 2, dans laquelle le phospholipide est la lécithine.

4. Composition selon la revendication 3, dans laquelle la quantité de diméthylisosorbide et de tétraglycol est de 8 à 30 % en poids par rapport au poids total de la composition.

5. Composition selon la revendication 4, dans laquelle le rapport de la quantité de diméthylisosorbide à la quantité de tétraglycol est de 2:1 à 1:3.

6. Composition selon la revendication 1, comprenant, en poids par rapport au poids total de la composition :
(a) de 0,1 à 0,6 % d'itraconazole ;
(b) de 10 à 30 % de phospholipide ;
(c) de 0,5 à 8 % de cholestérol ;
(d) de 3 à 10 % de diméthylisosorbide ;
(e) de 5 à 20 % de tétraglycol ;
(f) un tampon pour maintenir entre 5 et 7 le pH de la composition ;
(g) une quantité d'électrolyte suffisante pour stabiliser les membranes liposomiques ;
(h) une quantité de conservateurs acceptables d'un point de vue dermatologique, suffisante pour prévenir une dégradation de la composition ;
(i) de 0,5 à 1,5 % d'un agent épaississant ; et
(j) de l'eau.

7. Composition selon la revendication 6, dans laquelle
la quantité d'itraconazole est de 0,4 à 0,5 % ;
la quantité de phospholipide est de 15 à 25 % ;
la quantité de cholestérol est de 1,5 à 4 % ;
la quantité de diméthylisosorbide est de 3 à 7 % ;
la quantité de tétraglycol est de 5 à 15 % ; et
la quantité d'agent épaississant est de 1 %.

8. Composition selon la revendication 7, comprenant approximativement :
(a) 0,5 % d'itraconazole ;
(b) 20 % de phospholipide ;
(c) 2 % de cholestérol ;
(d) 5 % de diméthylisosorbide ;
(e) 10 % de tétraglycol ;
(f) 0,06 % d'hydroxyde de sodium et 0,1 % d'acide citrique ;
(g) 0,1 % de chlorure de sodium ;
(h) 0,2 % de méthylparaben, 0,02 % de propylparaben, 0,01 % d'hydroxytoluène butylé et 0,15 % d'édétate disodique ;
(i) 1 % d'hydroxypropylméthylcellulose ; et
(j) de l'eau.

9. Procédé pour préparer une composition selon l'une quelconque des revendications 1 à 8, caractérisé en ce que ledit procédé comprend les étapes consistant :
(a) à chauffer le phospholipide et le système solvant organique dans un premier récipient, à y dissoudre le principe actif, à ajouter les agents auxiliaires de formulation, et à agiter le mélange jusqu'à dissolution complète ;
(b) à chauffer une partie de l'eau dans un deuxième récipient et à y dissoudre les conservateurs, en laissant le mélange se refroidir, puis à ajouter le reste des agents auxiliaires de formulation et le reste de l'eau, et à agiter le mélange jusqu'à dissolution complète ; puis à préparer le constituant aqueux ;
(c) à transvaser le contenu du premier récipient, au moyen d'une dépression, directement dans le constituant aqueux, tout en homogénéisant la combinaison ainsi obtenue avec un mélangeur à grande vitesse, en particulier un mélangeur à grande vitesse de cisaillement ; et
(d) à ajouter un épaississant au mélange obtenu au moyen d'une dépression, tout en poursuivant l'homogénéisation.
